# EUROPEAN PATENT APPLICATION

(11) **EP 4 649 890 A1**
(43) Date of publication of application: **19.11.2025**
(21) Application number: 24175725.1
(22) Date of filing: 14.05.2024
(51) Int. Cl.: A61B 5/265, A61B 5/271, A61B 5/291, A61B 5/00, A61N 1/04, A61B 5/25, A61B 5/273

(54) **MEDICAL ELECTRODE**

(71) Applicant: Ambu A/S, 2750 Ballerup (DK)
(72) Inventor: SONNENBORG, Frederik Nikolaj Sværke, 4040 Jyllinge (DK)
(74) Representative: Winter, Brandl - Partnerschaft mbB

(57) **Abstract**

A medical electrode with a lead wire connector, the electrode including an electrode body with a skin side having a peripheral skin contact area and a central cavity with a central cavity area, both areas having an electrically conductive surface, the central cavity area being provided with at least one protrusion extending within the cavity only, and an outer side opposite to the skin side.

## Description

### TECHNICAL FIELD

The present disclosure relates to a medical electrode for measuring a biopotential, an electrode array comprising the medical electrode, and a method for manufacturing the electrode and electrode array.

### BACKGROUND

Electrodes are well-known devices in the medical field for patient check-ups and monitoring procedures in the health setting. Medical electrodes convey internal ionic current of the patient body into electrical current that can be amplified and evaluated for determining various conditions, disorders or diagnosis. Some of the main types of health examinations utilizing medical electrodes include electrocardiography (ECG) evaluating electrical activity of the heart, electroencephalograhy (EEG) measuring electrical waves of neuronal activity of the brain, polysomnygraphy (PSG), and electrophysiology (EP). EEG is the measurement of electrical potential differences across points on the scalp. These electrical potential differences are the result of electrical activation of diverse brain areas and are associated with brain function. The coherent activity of cortical pyramidal neurons generates ionic currents and these, in turn, give rise to an electric field and scalp electric potential. EEG opens a window into the functioning brain because neural function relies on electrochemical communication. The electric fields generated by the cortex provide a powerful, direct measure of its processes via EEG, which can record specific brain wave patterns in the patient. One example of a diagnostic medical electrode is the Ambu^{®} Neuroline^{™} Cup, which is a single-patient use cup electrode. Another example is discussed in WO 2021/099952 A1. Such cup electrodes may be attached to the scalp of a patient to detect neuronal activity of the brain, which is measured in µV.

Single-use electrodes optimize workflow and reduce cost while saving patient's lives and improving patient care. They optimize workflow and reduce cost because they are always ready when needed without the traditional large-scale capital and repair budgets required for reusable electrodes. For example, a sterilization and storage facility is avoided, there is no need to maintain evidence of sterilization, and there is no need to transport electrodes from sterilization and storage facilities to the buildings where they are needed, sometimes in the middle of the night or weekends. They save patient's lives and improve patient care because they are readily available and do not pose a cross-contamination risk. This also reduces hospital re-admissions. While single-use electrodes are disposed after a single patient use (one or more procedures may be performed while the patient remains in the treatment room), the environmental impact of re-useable electrodes, due to cleaning materials, CO₂ emissions during the cleaning process, and use of disposable personal protective equipment by personnel involved in transportation and sterilization of the re-useable electrodes, can be similar to that of single-use electrodes. To further reduce environmental impact, the electrodes according to the present disclosure are primarily made of polymer materials.

To further enhance the benefits of single-use medical electrodes, it is desirable to expand the applicability of the electrodes.

To further enhance the benefits of single-use medical electrodes, it is desirable to reduce manufacturing costs.

### SUMMARY

The present invention provides a medical electrode.
A first aspect relates to expanding the applicability of the electrodes. An embodiment of the first aspect relates to a medical electrode comprising: a lead wire connector, an electrode body including: a skin side; an outer side opposite the skin side; a central cavity having a height; and a rim surrounding the central cavity and having a thickness smaller than the height, the rim having a peripheral skin contact area and the central cavity having a central cavity area, each of the areas being on the skin side and having an electrically conductive surface, the central cavity area comprising at least one protrusion extending within the central cavity only. The protrusion increases the electrically conductive surface area, which increases the signal quality. Further the usable time of the electrode increases with the increased surface area. The usable time may be extended to a degree to enable long term monitoring ranging from a few hours to more than 24 hours, such as several days, e.g. 7 days. This may be advantageous when using the electrodes in an intensive care unit (ICU) setting and for examinations such as epilepsy and sleep studies, where monitoring for extended time is advantageous. Preparation of the patient, correct placement of the electrodes and correct set-up of the EEG system may take a trained health care professional considerable time, such as up to one hour, so replacement of electrodes is very time consuming and entails a risk that the electrodes are not positioned correctly. Enabling monitoring for extended periods without renewal of electrodes hence reduces the time and cost involved in providing EEG, so provision of an electrode usable for monitoring for extended time periods is advantageous.

In an variation of the present embodiment a medical electrode is provided, wherein the at least one protrusion is at least one rib arranged substantially radially, such as radial direction +/- 10deg, relatively to a central axis through the central cavity. The at least one rib is hence arranged substantially in a radial plane in relation to a central axis of the central cavity. Substantially radial direction is considered to include a deviation of +/- 10 deg, e.g. having a direction comprising a tangential component. The at least one protrusion could have other shapes, such as the shape of a pin or an annular rib, or a combination of shapes.

In another variation of the present embodiment the number of protrusions is below 10, such as in the range 2-8, for instance 3-5. A high number of protrusions provides some benefits, such as the posibility of a comparatively larger surface area, as well as the possibility of improved grip to possible gel or adhesive. However a high number of protrusions may increase manufacturing complexity and cost, and may result in reduced filling of the cavity in case a gel is used, which may negatively influence the performance of the electrode. At present three protrusions are considered a suitable compromise.

In another variation of the present embodiment the total active area of the electrode is in the range of 95 to 130 mm², such as 100 to 110 mm², for example 102 to 105 mm². The total active area is defined as the area of the electrode taking part in picking up signals. The total active area is considered to include the skin contact area and rounded edges thereof, if any, the cavity surface area and the protrusion surface area.

In another variation of the present embodiment the central cavity approximately has the shape of a dome or truncated cone. Other shapes are possible, such as a cylinder, a polygon prism or a cone. The dome or truncated cone is however considered an advantageous compromise when considering ergonomics for the patient and maximixing active surface area.

In another variation of the present embodiment the lead wire connector comprises a depression and at least one wall adjacent the depression.

In an example of this variation, the depression is sectioned by a step providing a shallow conducting portion and a deeper connection portion

Monitoring in neurological ICU's is often combined with (unplanned) Magnetic Resonance Imaging (MRI) or Computed Tomography (CT) scans. MRI uses strong magnetic fields (in the Radio Frequency range) to generate images of organs in the body, whereas CT scans use X-rays. For MRI the strong magnetic field may lead to possible reactions to metals introduced into the MR scanner. Traditional medical electrodes and electrode arrays are MR unsafe and should be removed before any MR scan, as MR unsafe parts could introduce a risk to the patient because of excessive heating of the parts. Further MR unsafe parts could deteriorate the imaging of MR or CT scans due to artifacts. Removal and refitting the electrodes is a tedious and time-consuming task. Providing a MR conditional electrode and electrode array hence expand the applicability of such electrodes and electrode arrays.

In another variation of the present embodiment the peripheral skin contact area comprises cut-outs in periphery, such as four cut-outs, with a depth in the range of 5-15 % of the diameter, such as 10% of the diameter, e.g. 0.5-1.5 mm, such as 1 mm in the case of a diameter of 10 mm. By depth is meant the radial extension of the cut-out from the periphery. The cut-outs may lower potential formation of eddycurrents in the electrode during an MR scan and hence lower the potential risk of artifacts on MRI.

In another variation of the present embodiment the electrode body comprises a polymer based core material with a conductive surface coating of Ag/AgCl. Hereby it is possible to provide an electrode with a relatively low environmetal impact in that the electrode contains very low amounts of metal, which is considered to have a relatively high environmental impact. Further such an electrode is suitable for mass production at relatively low cost, and hence particularly relevant for single-patient use. The low amount of metal also reduces the potential risk of artifacts in MR or CT scans.

In another variation of the present embodiment the cavity is prefilled with a conductive gel. Providing an electrode with prefilled cavity may simplify work for the health care specialist in that the electrode is ready to use without any further preparation, and is hence time saving. Downside is that the shelf life of the electrode may become shorter, so it is not always an advantage. Further the health care specialist may prefer a specific adhesive or gel for any given task, and in this case it is a disadvantage if the electrode is prefilled with another gel.

Another aspect of the disclosure relates to an electrode array comprising a plurality of medical electrodes as discussed above and a plurality of lead wires each comprising a lead wire conductor with an electrically insulating cladding, where each lead wire has a first end physically embedded in the lead wire connector and electrically connected to the electrically conducting surface of the electrode, and a second end connected to a connector. Hereby an electrode array may be provided having a very low amount of metal to the benefit of sustainability, and also lowering the risk of safety of image quality issues in MR or CT scans.

In a variation the lead wire conductor is made of an electrically conductive non-magnetic material, such as carbon or copper. Using a non-magnetic material lowers the risk of safety or image quality issues in MR or CT scans.

In a variation the length of lead wire is in the interval of 30 to 50 cm, such as 35 to 45 cm, such as approximately 40 cm. Such a lead wire length has hitherto been considered too long for MR conditional arrays, where standard lead wire length is approximately 25 cm. The lead wire may act as antenna in the magnetic field and cause heating, but the present inventor has found that it is a technical prejudice that the lead wire should have a length of 25 cm or less, and that a lead wire length in the given interval may indeed be considered MR conditional. A short lead wire length of e.g. 25 cm makes it difficult to correctly position the electrodes, and further the connector at the second end may in this case end up in a position, which is unpleasant for the patient and the relatively short lead wire length leaves very little room for adjustment of the connector position.

Another aspect of the invention relates to a method of making an electrode array as discussed above, the method comprising: providing an electrode body with a lead wire connector; positioning a lead wire in the lead wire connector; applying energy to material of the lead wire connector to soften or melt the material; allowing the softened or molten material to set around the lead wire, thereby integrating the lead wire in the lead wire connector of the medical electrode. This method simplifies the manufacturing process and reduces the manufacturing cost. Further with this method there is no need for mechanical anchoring or fixation by use of a metal crimp or anchoring and hence the risk of image artifacts in MR or CT scans is reduced.

One or more objects may be met by aspects of the present invention described in the following embodiments, variations and examples thereof.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

The disclosure will be described in more detail below with reference to the following figures. The figures illustrate embodiments, variations and examples to facilitate the understanding of a person of ordinary skill in the art and are not to be construed as limiting the scope of the invention.
FIG. 1 is a perspective illustration of an EEG system system including an electrode array, an electrode harness, a connector box and a computer;
FIG. 2 is an illustration of electrodes as applied to a patient,
FIG. 3 is a view from below of an embodiment of an electrode;
FIG. 4 is a top view of the electrode of FIG. 3;
FIG. 5 is a perspective top view of the electrode of FIG. 3,
FIG. 6 is a sectional side view of the electrode of FIG. 3;
FIG. 7A is a detail of FIG. 6 including a lead wire and a tool;
FIG. 7B corresponds to FIG. 7A with the lead wire integrated;
FIG. 8 is a perspective view from below of the electrode of FIG. 3,
FIG. 9 is a sectional side view of the electrode of FIG. 3 as attached to a patient;
FIG. 10 is a top view of an embodiment of another electrode;
FIG. 11 is a sectional side view of the electrode of FIG. 10;
FIG. 12 is a flowchart illustrating a method of making a medical electrode; and
FIG. 13 is a flowchart illustrating a method of making a medical electrode array.

### DETAILED DESCRIPTION OF THE DISCLOSURE

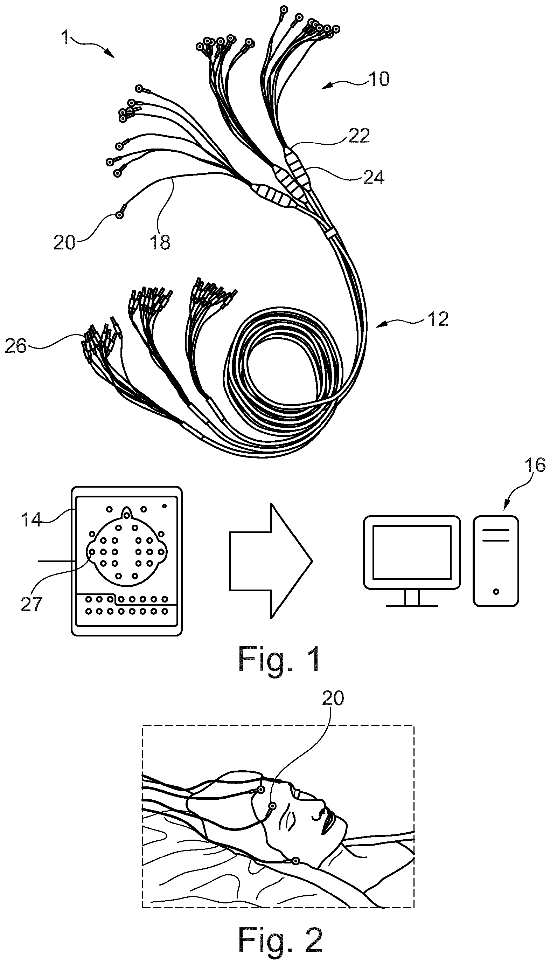

FIG. 1 illustrates an embodiment of an EEG system 1 including an EEG array 10, a harness 12, a connector box 14 and a processor 16. The EEG 10 array comprises several lead wires 18 each having a first end with an electrode 20 and a second end with an electrode connector 22. The harness 12 similarly has a first end with a harness connector 24 connectable to the electrode connector 22, and a second end with connectors, such as the illustrated harness jacks 26. The harness jacks 26 are in turn adapted to connect to the connector box 14 via sockets 27. The connector box in turn is adapted to connect to the processor 16 as schematically illustrated by an arrow. The connector box 14 and the processor 16 may be separate parts, but could alternatively be provided as one unit. The processor 16 is adapted to process input from the electrodes 20 and and output a signal that can be presented to a physician, such as in a graph on a screen or on print. The array 10 may be magnetic resonance (MR) conditional, meaning that under certain conditions it is safe to include the array 10 in an MR scan without a risk of harm to the patient and with low risk of negatively influencing image quality of the MR scan. For this the array 10 may be disconnected from the harness 12, which may be a reusable, MR unsafe part to lower cost and environmental impact, and in this event the harness 12 should not enter the MR room.

Electrodes as applied to a patient is illustrated in FIG. 2. The electrodes 20 are attached to the head of the patient, such as the scalp and forehead. Prior to application of the electrodes the skin of the patient is typically prepared to reduce the electrical resistance, such as by abrading the skin of the patient at the positions for electrode application. A gel, paste or adhesive may be used to attach the electrodes to the head of the patient. The gel, paste or adhesive may be electrically conductive, e.g. by containing chloride ions. As an example Ten20^{®} Conductive Paste by Weaver and Company may be used. It is also possible to use the electrodes in dry state without a gel, or to use e.g. a wet sponge with a saline solution. However, an electrically conductive gel is generally considered advantageous and may improve conduction and reduce skin-electrode interface impedance. Therefore, the gel between the skin and electrode allows for good-quality recording of biopotentials, which is measured in µV. Supplementary or alternatively, tape may be used to attach the electrodes to the head of the patient. In some cases an elastic electrode cap is used. An advantage of the elastic electrode cap is that application of the electrodes to the patient can be done very quickly. A disadvantage of the elastic electrode cap is that it may feel restrictive to the patient as the cap generally needs to be held down with straps anchored around the chin or chest. Further, such caps are generally not for single patient use and must hence be cleaned and dried after each use. Additional individual electrodes come at a lower cost and a versatile application area.

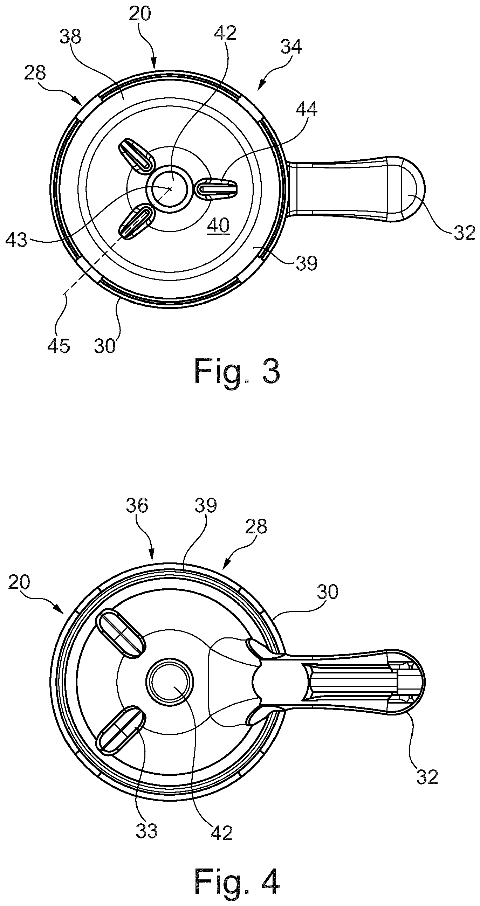

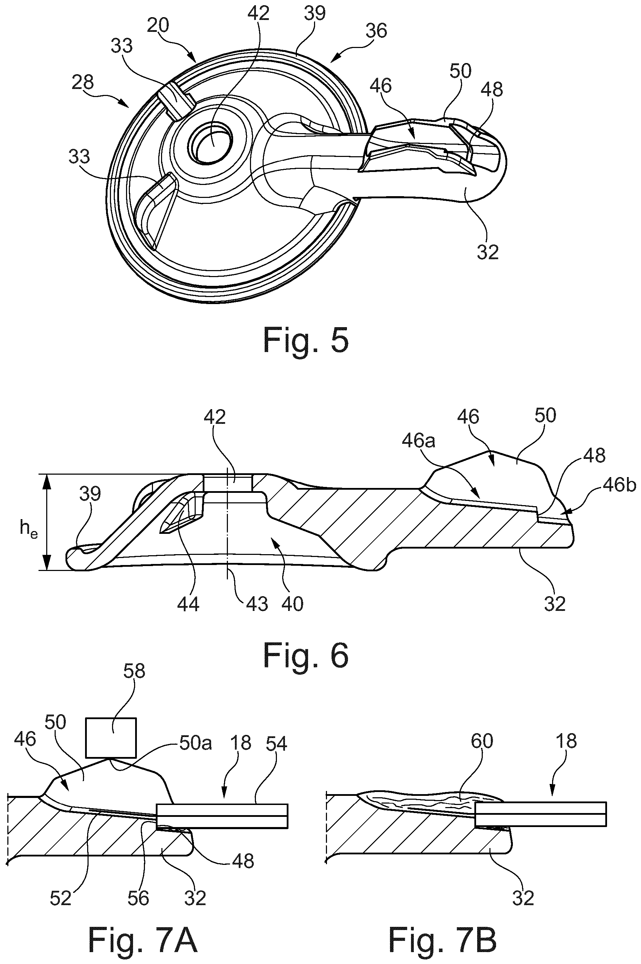

An embodiment of the electrode 20 is shown in FIGS. 3 to 9. The electrode body 28 has a skin side 34 (as illustrated in FIG. 3) adapted to face the skin of a patient, and an outer side 36 opposite the skin side (as illustrated in FIG. 4). Turning first to a view from below of FIG. 3, the illustrated electrode 20 has an electrode body 28 with a circular periphery 30 and a lead wire connector portion 32. The lead wire connector portion 32 is adapted to connect to the wire lead 18, as will be discussed in more detail below with reference to FIGS. 7A and 7B. The lead wire connector portion 32 of the illustrated embodiment may double as a mini-handle for the health care professional to ease handling and positioning of the electrode 20.

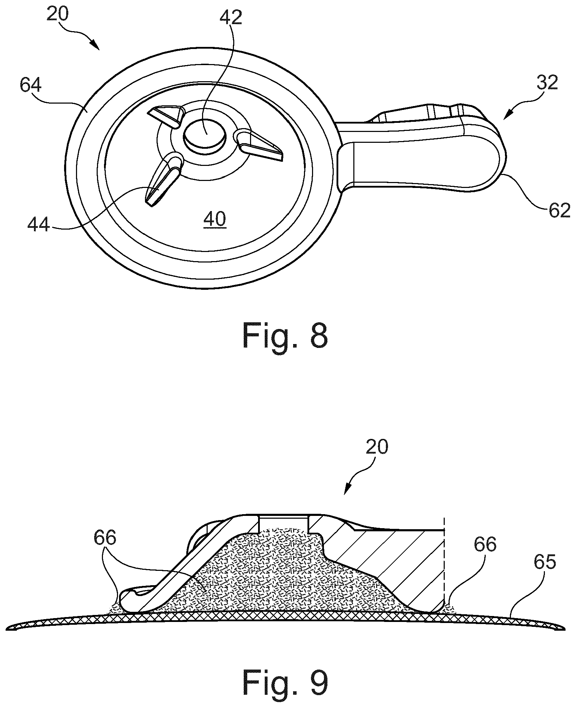

The electrode body comprises a rim 39 surrounding a central cavity 40. The rim 39 has a peripheral skin contact area 38 and the central cavity 40 extends outwardly from the skin contact area 38 such that a heigth of the central cavity 40 is greater than a thickness of the rim 39. The central cavity 40 has the approximate shape of a dome or truncated cone (see for example FIGS. 6 and 8). The central cavity 40 may, as illustrated, have an opening 42. The opening 42 allows for needle insertion and escape of air or surplus gel, if any. As an example, the opening may have a diameter in the interval of 1.5 to 2 mm. A central axis 43 intersects the opening 42 and extends through the central cavity 40, as also indicated on FIG. 6. Inside the central cavity 40, ribs 44 are arranged (see especially FIGS. 3, 6 and 8). The ribs 44 may be arranged in radial planes 45. The ribs 44 increase the surface area of the cavity's interior, and further may assist in holding any gel or paste in the cavity 40. The cavity of the electrode 20 may be prefilled with a gel or paste, thereby providing a ready-to-use electrode. Alternatively the electrode 20 may be supplied without gel or paste, which may be added to the electrode at a later stage, such as the time of application to the patient. The skin contact area 38 and the central cavity 40 has an electrically conductive surface. The electrode may or may not be made of a conductive material, whereas the surface should be conductive. The electrically conductive surface of this example comprises silver/silver chloride (Ag/AgCI), which is found to provide consistent and superior signal quality. Increasing the surface area improves signal quality and enables longer working periods of the electrodes. The ribs are sized and positioned so that they are contained inside the interior of the cavity 40. As the ribs do not extend outside of the interior of the cavity the ribs do touch the skin of the patient and hence do not negatively influence the patient's comfort.

For optimum patient comfort the peripheral skin contact area 38 preferably has curvatures having radius of at least 0.3 mm, such as at least 0.5 mm, at the transition from the skin contact area 38 to the interior cavity 40 at the inner periphery, and similarly at the outer periphery of the skin contact area 38. This is primarily of relevance for electrodes positioned so on a patients head where there is a risk that the electrode will be squeezed between the patient and a substrate. A height hₑ (shown in FIG. 6) of the electrode in the interval of 2.5 to 3.5 mm, such as 2.8 to 3.2, or such as 3 mm is found to provide a reasonable compromise between patient comfort and signal quality.

Referring now to FIG. 4, two fixation wings 33 are provided on the electrode body 28 for easier positioning (see also FIG. 5). The diameter of the electrode body in this embodiment is 10 mm, which is found to provide a reasonable compromise between size of the electrode and signal quality. If the electrode body has a much larger diameter it may be difficult to place electrodes correctly, especially when applied to children, whereas if the electrode is much smaller signal quality may become poor, and small electrodes may be difficult to handle for a health care professional.

FIGS. 5 and 6 illustrate features of the lead wire connector 32. The perspective top view of FIG. 5 illustrates the electrode 20 and specifically the second side 36 thereof with the wings 33, the opening 42 and the lead wire connector 32. FIG. 6 is a sectioned side view of the electrode 20. The lead wire connector 32 comprises a depression 46 comprising a step 48 as also illustrated in FIG. 6. The depression is in the illustrated embodiment bordered at two sides by walls 50. The step 48 sections the depression 46 in a shallow conducting portion 46a and a deeper connection portion 46b. The height hₑ of the electrode 20, described above, is also shown. Further FIG. 6 illustrate that the ribs 44 may be positioned at the same position as the the wings 33 and the lead wire connector 32. Superimposing the positions may be facilitate molding of the electrode 20 in view of flow of material in the mold.

FIGS. 7A and 7B are sectional views of the lead wire connector 32 in a process to connect a lead wire to the lead wire connector 32 of the electrode. FIG. 7A schematically illustrates a lead wire 18 with a lead wire conductor 52 having an insulating cladding 54. A portion of the lead wire 18 is stripped and the bare lead wire conductor 52 positioned in the conducting portion 46a of the depression 46 and with an unstripped portion of the lead wire positioned in the connection portion 46b with an end 56 of the insulating cladding positioned against the step 48. On top of the wall a tool 58 may be applied, such as an ultrasonic horn or a heat staking tool, to at least partially soften or melt the walls 50. The wall 50 comprises a peak 50a, which may aid in focusing the energy of the tool 58. The material of the walls redistribute as embedding material 60 to embed the lead wire in the lead wire connector 32 and electrically connect the lead wire conductor 52 to the electrode. This allows for automation and provides a secure connection without adding material. Other ways to connect the lead wire to the electrode are conceivable, such as using a crimp bushing or by soldering, which are simple and well known ways of connecting wires. However, these alternatives are less advantageous as they have some drawbacks. For example, the use of crimp bushings may result in faulty connection if the parts are not carefully arranged prior to application. Further, the electrical contact is not always optimum. Finally, such bushings are generally made of metal, which should be avoided for MR conditional electrodes. In case of soldering, cold solder joints may occur, creating an incorrect joint. Cold solder joints are not always easy to detect and may result in faulty electrodes. Further, the use of solder adds metal to the electrode, which should be avoided for MR conditional electrodes.

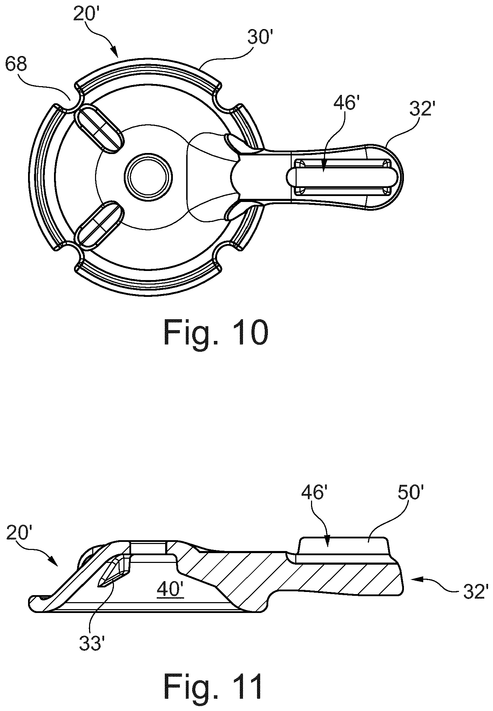

In a variation of the present embodiment, the electrode 20 comprises peripheral cut-outs 68 described below with reference to FIGS. 10 and 11.

In a further variation of the present embodiment, the electrode 20 comprises a simplified lead wire connector 32' described below with reference to FIGS. 10 and 11.

In a yet further variation of the present embodiment, the electrode 20 comprises the peripheral cut-outs 68 and the simplified lead wire connector 32'.

FIG. 8 provides a view of the first side of the electrode showing the interior cavity 40, the opening 42 and the ribs 44. The lead wire connector 32 advantageously has a rounded end 62 to limit any potential discomfort for a patient should the lead wire connector 32 get in contact with the skin of the patient. Similarly the outer edge 64 of the electrode 20 preferably has a rounded profile to minimize any potential discomfort for the patient.

FIG. 9 schematically illustrates the electrode's position on the skin 65 of a patient. Here a conductive gel 66 is applied to the electrode to attach the electrode to the skin 65, and to lower the skin-electrode interface impedance.

An alternative embodiment of the electrode is illustrated in FIGS. 10 and 11. This embodiment shares many features of the previous embodiment, but it also has some differences, which will be discussed in the following. When comparing FIG. 10 of this alternative embodiment with the similar view of the first embodiment in FIG. 4 the most apparent difference is the provision of cut-outs 68 in the periphery of the electrode 20'. The cut-outs 68 minimize the potential formation of eddy currents in the electrode 20' when subjected to a changing magnetic field, such in an MR scanner. Eddy currents could potentially lead to artifacts on the MR image, so with the cut-outs 68 an electrode 20' is provided with reduced risk of artifacts in MR imaging. The diameter of the electrode 20' could be around 10 mm. The risk of eddy current formation and artifacts increase with increasing diameter, so the effect of providing cut-outs 68 will be more pronounced for larger electrodes. Here a total of four cut-outs are provided, but one cut-out 68 could in some cases be enough, e.g. for electrodes having a small diameter, or if there is no specific need for artifact-free images. If more than one cut-out is provided they should preferably have equal spacing to provide the shortest possible unbroken section of the periphery. Having more than five cut-outs is generally not recommended for the electrodes as the active area of the electrode is considered to be reduced too much, and further provision of cut-outs complicates molding. The illustrated cut-outs are rounded, but could alternatively be slots or V-shaped cut-outs. Rounded cut-outs are however presently preferred to limit the risk of sharp edges, which could give rise to discomfort for the patient. The peripheral cut-outs 68 can also be provided in the embodiment of the electrode 20 shown in FIGS. 3 to 7B.

Another difference of the alternative embodiment relates to the lead wire connector which can be seen when comparing the sectional view of FIGS. 6 and 11. The lead wire connector 32' of the alternative embodiment comprises a simplified wall 50' having a straight upper edge without a peak, making the electrode slightly simpler to produce. A lead wire (not shown) may be positioned in the depression 46' and embedded in material in a similar way as exemplified above. As shown, the step 48 is omitted. In another example the step 48 is provided. Further, the lead wire connector 32' can be provided in the embodiment of the electrode 20 shown in FIGS. 3 to 7B.

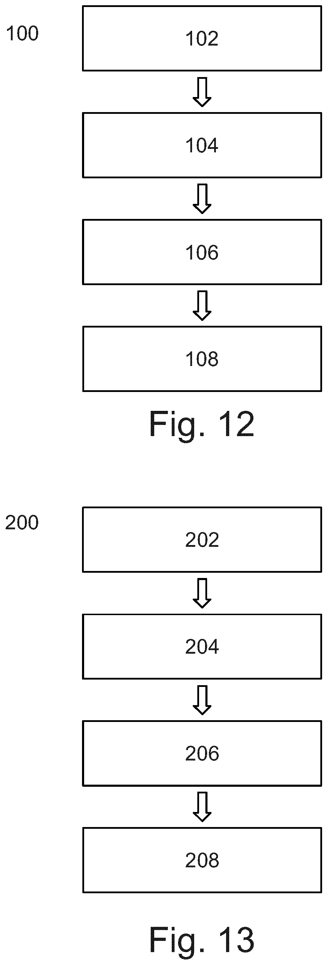

Another aspect of the disclosure relates to a method of making a medical electrode as discussed above, which will now be described with reference to a flowchart 100 in FIG. 12. In an embodiment of the method of making medical electrodes, the method comprises: at 102, providing a mould with protrusion forming recesses; at 104, injecting a polymer into the mould; at 106, ejecting the electrode blank from the mould; and at 108 coating the electrode blank with an electrically conductive surface layer, such as Ag/AgCl. The polymer may for example be polycarbonate (PC) or Acrylonitrile Butadiene Styrene (ABS) or a mixture of PC and ABS, potentially with reinforcing fibres, such as carbon fibres.

A further aspect of the disclosure relates to a method of making an electrode array as discussed above, which will now be described with reference to a flowchart 200 in FIG. 13. In an embodiment of the method of making medical electrodes, the method comprises: at 202, providing an electrode body with a lead wire connector; at 204, positioning a lead wire in the lead wire connector; at 206, applying energy to material of the lead wire connector to soften or melt the material; at 208, allowing the softened or molten material to set around the lead wire, thereby integrating the lead wire in the lead wire connector of the medical electrode.

### Parts list:

- 1: EEG system
- 10: EEG array
- 12: harness
- 14: connector box
- 16: processor
- 18: lead wire
- 20,20': electrode
- 22: electrode connector
- 24: harness connector
- 26: jack
- 27: socket
- 28: electrode body
- 30: periphery
- 32: lead wire connector
- 33: fixation wing
- 34: skin side
- 36: outer side
- 38: skin contact area
- 39: rim
- 40: central cavity
- 42: opening
- 43: central axis
- 44: rib
- 45: radial plane
- 46: depression
- 46a: conducting portion
- 46b: connection portion
- 48: step
- 50: wall
- 52: lead wire conductor
- 54: insulating cladding
- 56: end
- 58: tool
- 60: embedding material
- 62: rounded end
- 64: outer edge
- 65: skin
- 66: gel
- 68: cut-out
- 100: flowchart
- 200: flow-chart
- hₑ: electrode height

## Claims

1. A medical electrode, comprising:
a lead wire connector, and
an electrode body including:
a skin side;
an outer side opposite the skin side;
a central cavity having a height; and
a rim surrounding the central cavity and having a thickness smaller than the height, the rim having a peripheral skin contact area and the central cavity having a central cavity area, each of the areas being on the skin side and having an electrically conductive surface, the central cavity area comprising at least one protrusion extending within the central cavity only.

2. Medical electrode of claim 1, wherein the at least one protrusion is at least one rib arranged substantially radially, such as radial direction +/- 10deg, relatively to a central axis through the central cavity.

3. Medical electrode of any one of the claims above, wherein the number of protrusions is below 10, such as in the range 2-8, for instance 3-5.

4. Medical electrode of any one of the claims above, wherein the electrode has a total active area in the range of 95 to 130 mm², such as 100 to 110 mm², for example 102 to 105 mm².

5. Medical electrode of any one of the claims above, wherein the central cavity approximately has the shape of a dome or truncated cone.

6. Medical electrode of any one of the claims above, wherein the lead wire connector comprises a depression and at least one wall adjacent the depression.

7. Medical electrode of claim 6, the depression is sectioned by a step providing a shallow conducting portion and a deeper connection portion.

8. Medical electrode of any one of the claims above, wherein the peripheral skin contact area comprises cut-outs in periphery (clover), such as four cut-outs, each having a depth in the range of 0.5-1.5 mm, such as 1 mm.

9. Medical electrode of any one of the claims above, wherein the body comprises a polymer-based core material with a conductive surface coating of Ag/AgCl.

10. Medical electrode of any of the claims above, wherein the cavity is prefilled with a conductive gel.

11. Electrode array comprising a plurality of medical electrodes of claim 1 and a plurality of lead wires each comprising a lead wire conductor with an electrically insulating cladding, where each lead wire has a first end physically embedded in the lead wire connector and electrically connected to the electrically conducting surface of the electrode, and a second end connected to an electrode connector.

12. Electrode array of claim 11, wherein the lead wire conductor of each lead wire is made of an electrically conductive non-magnetic material, such as carbon or copper.

13. Electrode array of claim 11 or 12, wherein each lead wire has a length in the interval of 30 to 50 cm, such as 35 to 45 cm, such as approximately 40 cm.

14. A method of making an electrode array, the method comprising: providing an electrode body with a lead wire connector; positioning a lead wire in the lead wire connector; applying energy to material of the lead wire connector to soften or melt the material; allowing the softened or molten material to set around the lead wire, thereby integrating the lead wire in the lead wire connector of the medical electrode.
